(19) Europäisches Patentamt
European Patent Office
Office européen des brevets

(11) **EP 3 104 163 B1**

(12) **EUROPÄISCHE PATENTSCHRIFT**

(45) Veröffentlichungstag und Bekanntmachung des Hinweises auf die Patenterteilung:
**20.12.2017 Patentblatt 2017/51**

(21) Anmeldenummer: **15171901.0**

(22) Anmeldetag: **12.06.2015**

(51) Int Cl.:
*G01N 21/3504* *(2014.01)*  *G01N 21/15* *(2006.01)*
*G01N 21/27* *(2006.01)*  *G01N 21/85* *(2006.01)*
*G01N 33/00* *(2006.01)*

(54) **PROZESS-GASANALYSATOR UND VERFAHREN ZUR ANALYSE EINES PROZESSGASES**

PROCESS GAS ANALYSER AND METHOD FOR ANALYSING A PROCESS GAS

ANALYSEUR DE GAZ DE PROCESSUS ET PROCÉDÉ D'ANALYSE D'UN GAZ DE PROCESSUS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**14.12.2016 Patentblatt 2016/50**

(73) Patentinhaber: **Siemens Aktiengesellschaft 80333 München (DE)**

(72) Erfinder: **Bitter, Ralf 76139 Karlsruhe (DE)**

(56) Entgegenhaltungen:
**EP-A1- 1 693 665    US-A1- 2012 236 323**

**Beschreibung**

Die Erfindung betrifft einen Prozess-Gasanalysator nach dem Oberbegriff des Anspruches 1. Die Erfindung betrifft ferner ein Verfahren nach dem Oberbegriff des Anspruches 5.

Ein derartiger Prozess-Gasanalysator bzw. ein derartiges Verfahren sind z. B. aus der US 2012/0236323 A1 bekannt. Bei nach dem Durchlichtverfahren arbeitenden Gasanalysatoren wird das Licht einer Lichtquelle durch das zu analysierende Gas geleitet und anschließend detektiert. Das Licht kann wellenlängenselektiv erzeugt und breitbandig detektiert werden (z. B. Laserspektrometer), oder es kann breitbandig erzeugt und wellenlängenselektiv detektiert werden (z. B. nicht-dispersiver Infrarot (NDIR)-Gasanalysator). Bei in-situ Prozess-Gasanalysatoren, wie sie aus der US 2012/0236323 A1, der DE 10 2013 213 730 A1 oder der EP 1 693 665 A1 bekannt sind, sind die Lichtquelle und der Detektor üblicherweise in unterschiedlichen Messköpfen untergebracht, die an Prozessflanschen auf einander diametral gegenüberliegenden Seiten eines das zu messende Prozessgas enthaltenden oder führenden Anlagenteils (z. B. Abgasleitung, Behälter, Kamin) montiert sind. Damit die Lichtquelle und der Detektor nicht in Kontakt mit dem oft aggressiven, heißen und staubhaltigen Prozessgas kommen, sind sie hinter Fenstern angeordnet. Das Fenster schließt ein Ende eines Spülrohres ab, das mit seinem anderen offenen Ende in das gasführende Anlagenteil mündet und mit einem Spülgas bespült wird. Das Spülgas wird so gewählt, dass es ohne Quergaseinfluss auf die jeweils zu messenden Gaskomponenten ist, d. h. seine spektralen Absorptionslinien liegen außerhalb der für die Messung genutzten Absorptionslinien des Prozessgases. Das Spülgas tritt aus den offenen Enden der einander gegenüberliegenden Spülrohre aus, so dass die Messstrecke für die Absorptionsmessung des Prozessgases durch den Abstand der offenen Enden beider Spülrohre bestimmt wird.

Je höher der Spülgasdurchfluss ist, umso wirkungsvoller gelingt es, die Fenster von Verunreinigungen aus dem Prozessgas freizuhalten. Dabei können die Spülraten je nach Applikation im Bereich von wenigen Litern in der Minute bis hin zu mehreren hundert Litern in der Minute variieren. Ein hoher Spülgasverbrauch ist bei Verwendung von Flaschengas jedoch mit entsprechend hohen Kosten verbunden. So wird beispielsweise für die Messung von Sauerstoff oft Stickstoff als Spülgas benutzt. In Fällen, in denen Umgebungsluft als Spülgas geeignet ist, können variable Feuchtegehalte zu Quereinflüssen mit zu messenden Gaskomponenten führen.

Da die Gasanalyse auf der spezifischen Lichtabsorption der zu messenden Gaskomponente beruht und die Absorption von dem Produkt aus der Konzentration der Gaskomponente und der Absorptionsstrecke abhängt bzw. bei geringen Konzentrationen näherungsweise proportional dazu ist, wird die Messung durch das in die Messstrecke zwischen den einander gegenüberliegenden Spülrohren einströmende und dort teilweise das Prozessgas verdrängende und sich mit ihm vermischende Spülgas gestört. Außerdem kann das einströmende Spülgas Parameter wie Druck, Strömung und Temperatur des Prozessgases verändern, welche die Lichtabsorption beeinflussen. Dies führt im Ergebnis dazu, dass die effektive Absorptionsstrecke (Messstrecke) in dem zu messenden Prozessgas nicht mit dem Abstand der offenen Enden der beiden Spülrohre übereinstimmt, sondern in unbekanntem Maße davon abweicht und variieren kann.

Bisher wurde der durch die Spülung verursachte Messfehler dadurch reduziert, dass für eine konstante Prozesskonstellation ein Korrekturfaktor für die Änderung der effektiven Messstrecke und/oder ein Offset für einen möglichen Konzentrationseinfluss des Spülgases bestimmt wurde. Dies funktioniert nur, solange die Prozess- und Spülbedingungen (Spülgaskonzentration, Druck, Temperatur, Volumenstrom) konstant sind.

Bei dem aus der eingangs erwähnten US 2012/0236323 A1 bekannten Prozess-Gasanalysator werden die Spülrohre mittels eines schaltbaren Ventils kurzzeitig von der Spülgaszufuhr getrennt und anschließend vollständig mit Prozessgas gefüllt, indem mittels einer Pumpe oder eines Gebläses anstelle des Spülgases das Prozessgas in die Spülrohre eingeleitet wird oder in den Spülrohren vorhandene Spülgas abgesaugt und von dem nachströmenden Prozessgas ersetzt wird. Die zur Ermittlung der Konzentration der zu messenden Gaskomponente relevante effektive Absorptionsstrecke (Messstrecke) wird dadurch bestimmt, dass der bekannte Abstand zwischen Lichtquelle und Detektor mit dem Verhältnis der bei Füllung der Spülrohre einmal mit dem Spülgas und das andere Mal mit dem Prozessgas jeweils detektierten Absorptionen multipliziert wird. Der Einfluss der Absorption durch das Spülgas auf die Messung bleibt dabei unberücksichtigt. Das Prozessgas kommt in Kontakt mit den die Lichtquelle und den Detektor schützenden Fenstern. Der Vorgang des Füllens der Spülrohre mit dem Prozessgas und anschließenden Wiederbefüllens mit dem Spülgas kann bei Bedarf wiederholt erfolgen, unterbricht aber jedes Mal die laufende Messung.

Aus der EP 1 693 665 A1 ist es bekannt, den Einfluss der Absorption durch das Spülgas auf die Analyse des Prozessgases dadurch zu kompensieren, dass das Spülgas nach Durchströmen der Spülrohre aus diesen abgezogen und in einem separaten Messkanal analysiert wird. Das Ergebnis der Spülgasanalyse wird von dem Ergebnis der Prozessgasanalyse subtrahiert. Für den separaten Messkanal wird ein Teil des für die Analyse des Prozessgases erzeugten Lichts abgezweigt und nach Durchstrahlen einer von dem gesammelten Spülgas durchströmten Messküvette separat detektiert. Der konstruktive Aufwand ist daher entsprechend groß.

Der Erfindung liegt daher die Aufgabe zugrunde, eine weitgehende Kompensation des durch die Spülung verursachten Messfehlers auch bei unterschiedlichen und sich verändernden Prozessbedingungen zu ermöglichen und größere Freiheiten bei der Wahl des Spülgases zu erlauben, was zu einer Kostenersparnis bei dem Betrieb des Gasanalysators führt.

Gemäß der Erfindung wird die Aufgabe durch den in Anspruch 1 definierten Prozess-Gasanalysator bzw. das in Anspruch 5 angegebene Verfahren gelöst.

Vorteilhafte Weiterbildungen der Erfindung sind in den Unteransprüchen angegeben.

Die Erfindung sieht also vor, den Volumenstrom des Spülgases zu modulieren, d. h. während der laufenden Messung bzw. Analyse periodisch zu ändern. Der Volumenstrom kann in Schritten, z. B. rechteck- oder treppenförmig, oder kontinuierlich, z. B. sinus- oder dreieckförmig variiert werden. Anhand von durch die Modulation hervorgerufenen, d. h. mit ihr korrelierenden Änderungen der detektierten Absorption wird der Einfluss des Spülgases auf das Analysenergebnis bestimmt und aus diesem entfernt. Bei schrittweiser Veränderung des Volumenstroms werden bei jedem Schritt die Änderungen der detektierten Absorption ermittelt und ausgewertet. Bei kontinuierlicher Veränderung des Volumenstroms werden die daraus resultierenden Änderungen der detektierten Absorption vorzugsweise frequenzselektiv bei der Modulationsfrequenz und/oder Oberschwingungen der Modulation ermittelt und ausgewertet, beispielsweise unter Verwendung eines Lock-in Algorithmus.

Die Modulation des Volumenstroms des Spülgases kann in einfacher Weise mittels eines drehzahlvariablen Gebläses zur Förderung des Spülgases oder eines steuerbaren Regelventils in der Spülgaszufuhr erfolgen.

Zur weiteren Erläuterung der Erfindung wird im Folgenden auf die Figuren der Zeichnung Bezug genommen; im Einzelnen zeigen

Figur 1    ein Ausführungsbeispiel des erfindungsgemäßen Prozess-Gasanalysators und

Figur 2    ein Beispiel für eine Kalibration des erfindungsgemäßen Prozess-Gasanalysators.

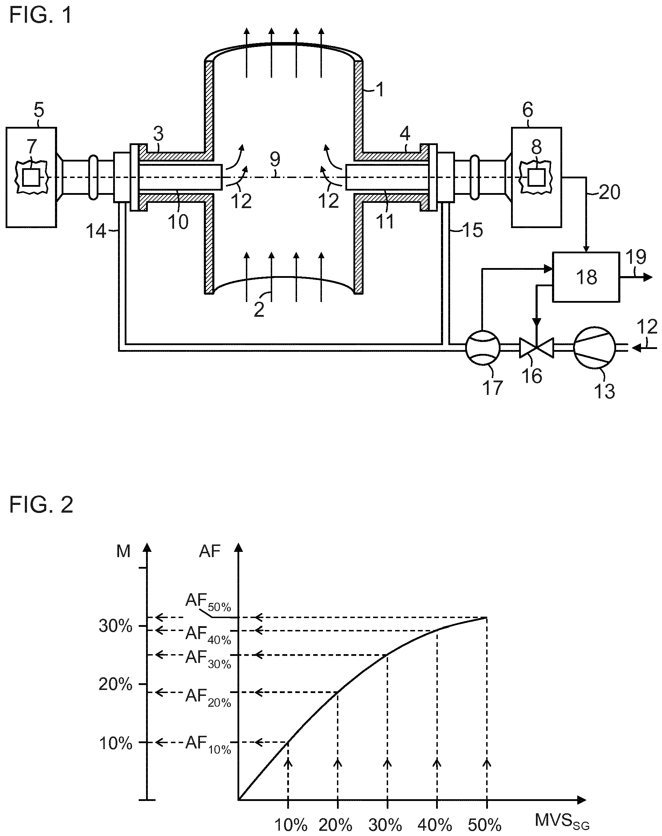

Figur 1 zeigt in schematischer Darstellung ein Anlagenteil 1, z. B. einen Abgaskanal, durch den ein zu analysierendes Prozessgas 2 strömt. Das Anlagenteil 1 weist an zwei diametral gegenüberliegenden Stellen Prozessflansche 3, 4 auf, an denen zwei im Wesentlichen baugleiche Messköpfe 5, 6 eines Prozess-Gasanalysators montiert sind. Beide Messköpfe 5, 6 enthalten jeweils ein optoelektronisches Element 7, 8, das in dem einen Fall eine Lichtquelle 7, z. B. eine Laserdiode, und in dem anderen Fall ein Detektor 8, z. B. ein Photodetektor, ist. Das von der Lichtquelle 7 erzeugte Licht 9 wird durch das Anlagenteil 1 mit dem darin geführten Prozessgas 2 geleitet und trifft anschließend auf den Detektor 8. Die optoelektronischen Elemente 7, 8 sind durch hier nicht gezeigte Fenster von dem Inneren des Anlagenteils 1 und damit von dem Prozessgas 2 getrennt, wobei zwischen den Fenstern und dem Inneren des Anlagenteils 1 zwei Spülrohre 10, 11 vorgesehen sind, die an einem Ende mit dem jeweiligen Fenster abgeschlossen sind und mit ihrem anderen offenen Ende in das Innere des Anlagenteils 1 münden. Die Spülrohre 10, 11, durch die das Licht 9 verläuft, werden von einem Spülgas 12 durchspült, das von einem Gebläse 13 gefördert und über Spülgaszuführungen 14, 15 jeweils in Fensternähe in die Spülrohre 10, 11 eingeleitet wird und diese an ihren offenen Enden verlässt. In der gemeinsamen Spülgaszufuhr sind ein steuerbares Regelventil 16 und ein Durchflussmesser 17 angeordnet. Alternativ können in jeder der beiden Spülgaszuführungen 14, 15 jeweils ein steuerbares Regelventil und ein Durchflussmesser vorhanden sein. Auch kann für jede der beiden Spülgaszuführungen 14, 15 jeweils ein eigenes Gebläse vorgesehen werden.

Dem Detektor 8 ist eine Auswerteeinrichtung 18 nachgeordnet, der die wellenlängenspezifische Absorption des Lichts 9 in dem Prozessgas 2 zu einem Analysenergebnis 19 auswertet. Typischerweise handelt es sich bei dem Gasanalysator um ein Laserspektrometer, bei dem, wie beispielsweise aus der DE 10 2012 223 874 B3 bekannt, die Wellenlänge des erzeugten Lichts 9 auf eine spezifische Absorptionslinie einer zu messenden Gaskomponente des Prozessgases 2 abgestimmt und dabei die Absorptionslinie periodisch wellenlängenabhängig abgetastet wird. Während der vergleichsweise langsamen Abtastung der Absorptionslinie kann zusätzlich die Wellenlänge des Lichts mit hoher Frequenz und kleiner Amplitude sinusförmig moduliert werden. Das von dem Detektor 8 erzeugte Messsignal 20 wird direkt oder nach Demodulation bei einer n-ten Harmonischen der Modulationsfrequenz ausgewertet. Die Auswertung erfolgt z. B. durch Anfitten des Lorentz-Profils einer idealen Absorptionslinie bzw. dessen n-ten Ableitung an den Verlauf des (ggf. demodulierten) Messsignals. Aus dem dabei erhaltenen Messergebnis wird schließlich die Konzentration der zu messenden Gaskomponente als das Analysenergebnis 19 bestimmt.

Um, wie im Folgenden an einem Beispiel näher erläutert wird, den Einfluss des Spülgases 12 auf das Analysenergebnis 19 zu minimieren, wird der Volumenstrom des Spülgases 12 mit Hilfe des Regelventils 16 oder alternativ des Gebläses 13 moduliert. Die Modulation wird durch die Auswerteeinrichtung 18 gesteuert, die anhand des gemessenen Durchflusses den Modulationsgrad auf einen vorgegebenen prozentualen Wert regelt.

Die wellenlängenabhängige Intensitätsabnahme des Lichts 9 auf dem Weg von der Lichtquelle 7 zu dem Detektor 8 wird durch das Lambert-Beersche Gesetz beschrieben:

$$I = I_0 \cdot exp(- \sigma_{MG} \cdot l_{MG} \cdot c_{MG} - \sigma_{SG} \cdot l_{SG} \cdot c_{SG}).$$

Dabei sind an der Stelle (Wellenlänge) der interessierenden Absorptionslinie der zu messenden Gaskomponente (Messgas):

| | |
|---|---|
| I | die detektierte Lichtintensität, |
| $I_0$ | die Ausgangsintensität des von der Lichtquelle 7 ausgestrahlten Lichts, |
| $\sigma_{MG}$ | der Absorptionskoeffizient des Messgases, |
| $l_{MG}$ | die Messstrecke in dem Prozessgas, |
| $c_{MG}$ | die Konzentration des Messgases, |
| $\sigma_{SG}$ | der Absorptionskoeffizient des Spülgases, |
| $l_{SG}$ | die von dem Spülgas bespülte Absorptionsstrecke und |
| $c_{SG}$ | die Konzentration des Spülgases. |

Mit der Gesamtabsorptionsstrecke $l_0 = l_{MG} + l_{SG}$ erhält man:

$$I = I_0 \cdot exp(- \sigma_{MG} \cdot (l_0 - l_{SG}) \cdot c_{MG} - \sigma_{SG} \cdot l_{SG} \cdot c_{SG}).$$

Bei einer sinusförmigen Modulation des Spülgasstromes ändert sich die von dem Spülgas bespülte Absorptionsstrecke $l_{SG}$ entsprechend:

$$l_{SG} = l_{SG0} \cdot (1 + M \cdot sin\, 2\pi ft),$$

wobei M (0 < M < 1) die normierte Amplitude und f die Frequenz der Modulation ist.
Dementsprechend ergibt sich für die detektierte Lichtintensität:

$$I = I_0 \cdot exp(- \sigma_{MG} \cdot l_0 \cdot c_{MG} + ( \sigma_{MG} \cdot c_{MG} - \sigma_{SG} \cdot c_{SG}) \cdot l_{SG0} \\ + ( \sigma_{MG} \cdot c_{MG} - \sigma_{SG} \cdot c_{SG}) \cdot l_{SG0} \cdot M \cdot sin\, 2\pi ft).$$

Zur rechnerischen Vereinfachung wird im Folgenden die Extinktion oder Absorbanz auf Basis des natürlichen Logarithmus verwendet:

$$E = - ln\frac{I}{I_0} = \sigma_{MG} \cdot l_0 \cdot c_{MG} - ( \sigma_{MG} \cdot c_{MG} - \sigma_{SG} \cdot c_{SG}) \cdot l_{SG0} \\ -( \sigma_{MG} \cdot c_{MG} - \sigma_{SG} \cdot c_{SG}) \cdot l_{SG0} \cdot M \cdot sin\, 2\pi ft.$$

Die detektierte Extinktion E hat also neben einem Gleichanteil einen Wechselanteil mit der Amplitude AF:

$$AF = ( \sigma_{MG} \cdot c_{MG} - \sigma_{SG} \cdot c_{SG}) \cdot l_{SG0} \cdot M.$$

$$AF = ( \sigma_{MG} \cdot c_{MG} - \sigma_{SG} \cdot c_{SG}) \cdot l_{SG0} \cdot M.$$

Damit lässt sich die Gleichung für die detektierte Extinktion E wie folgt umschreiben:

$$E = \sigma_{MG} \cdot l_0 \cdot c_{MG} - \frac{AF}{M} - AF \cdot sin\, 2\pi ft.$$

Die detektierte Extinktion E besteht also aus einem von dem Spülgas unbeeinflussten ersten Gleichanteil $\sigma_{MG} \cdot l_0 \cdot c_{MG}$, einem von dem Spülgas beeinflussten zweiten Gleichanteil AF/M und dem Wechselanteil mit der Amplitude AF.

Wird der Volumenstrom des Spülgases 12 um einen vorgegebenen niedrigen Prozentsatz, beispielsweise um 10%, variiert, wird sich mit hinreichender Näherung auch die von dem Spülgas bespülte Absorptionsstrecke $l_{SG}$ um denselben Prozentsatz ändern, d. h. die normierte Amplitude M wird den Wert 0,1 haben. Da die Amplitude AF des Wechselanteils durch Auswertung der Extinktion E bei der Modulationsfrequenz f unmittelbar ermittelt werden kann, ist auch der oben erwähnte zweite Gleichanteil AF/M bekannt, hier AF/M = AF/0,1 = 10·AF. Schließlich sind auch die Gesamtabsorptionsstrecke $l_0$ und der Absorptionskoeffizient $\sigma_{MG}$ des Messgases bekannte Größen, so dass die Konzentration $c_{MG}$ des Messgases frei von Einflüssen des Spülgases 12 aus der detektierten Extinktion E bzw. der detektierten Lichtintensität I bestimmt werden kann.

Bei größeren Änderungen des Volumenstroms des Spülgases 12 wird sich die von dem Spülgas bespülte Absorptionsstrecke $l_{SG}$ nicht linear, d. h. nicht um denselben Prozentsatz ändern. In diesem Fall wird die normierte Amplitude M, mit der sich die von dem Spülgas bespülte Absorptionsstrecke $l_{SG}$ ändert, im Rahmen einer einmaligen Kalibrierung in Abhängigkeit von unterschiedlich großen Änderungen des Volumenstroms des Spülgases 12 ermittelt. Wie Figur 2 verdeutlicht, können zu diesem Zweck bei konstanten Prozessbedingungen für unterschiedliche normierte Modulationsamplituden $MVS_{SG}$ des Spülgasstromes, hier z. B. $MVS_{SG}$ = 10%, 20%, ... 50%, die zugehörigen der detektierten Extinktion E ermittelt werden. Nun kann M einfach mit

$$M = \frac{AF \cdot x}{AF_x}$$

ermittelt werden, wobei x eine möglichst kleine relative Modulationsamplitude bezeichnet, so dass gilt: $MVS_{SG}$ = M = x. Mit x = 10% ergibt sich beispielsweise bei einer Modulation des Spülgasstromes mit $MVS_{SG}$ = 50% eine resultierende Modulation M der von dem Spülgas bespülten Absorptionsstrecke $l_{SG}$ von:

$$M = \frac{AF_{50\%} \cdot 0.1}{AF_{10\%}}.$$

Nach der beschriebenen Kalibrierung lässt sich das erfindungsgemäße Verfahren mit beliebiger Modulation des Volumenstroms des Spülgases 12 durchführen, auch wenn der Zusammenhang zwischen M und $MVS_{SG}$ nicht linear ist.

Sich ändernde Prozessbedingungen wie Druck, Temperatur oder Volumenstrom des Prozessgases 2 in dem Anlagenteil 1, beeinflussen zwar die von dem Spülgas 12 bespülte Absorptionsstrecke $l_{SG}$, werden aber durch das erfindungsgemäße Verfahren weitgehend kompensiert. Wenn sich beispielsweise der Druck in dem Anlagenteil 1 erhöht, verringert sich die von dem Spülgas bespülte Absorptionsstrecke $l_{SG}$, wobei sich in erster Näherung die modulationsbedingte Änderung von $l_{SG}$ in gleichem Maße mitändert und M somit konstant bleibt.

Wie bereits erwähnt, kann der Volumenstrom des Spülgases 12 in nahezu beliebiger Weise moduliert werden, um anschließend anhand der durch die Modulation hervorgerufenen Variationen der detektierten Absorption den Einfluss des Spülgases 12 auf das Analysenergebnis 19 zu bestimmen und aus diesem herauszurechnen. Beispielsweise bewirkt eine rechteckförmige Modulation des Volumenstroms des Spülgases 12, dass die von dem Spülgas bespülte Absorptionsstrecke $l_{SG}$ periodisch zwischen den Werten $l_{SG1}$ = $l_{SG0}$ und $l_{SG2}$ = $l_{SG0} \cdot (1+M)$ wechselt. Entsprechend erhält man für die detektierte Extinktion E in jeder Modulationsperiode zwei Werte E1 und E2:

$$E1 = \sigma_{MG} \cdot l_0 \cdot c_{MG} - (\sigma_{MG} \cdot c_{MG} - \sigma_{SG} \cdot c_{SG}) \cdot l_{SG0}$$

und

$$E2 = \sigma_{MG} \cdot l_0 \cdot c_{MG} - (\sigma_{MG} \cdot c_{MG} - \sigma_{SG} \cdot c_{SG}) \cdot l_{SG0}$$
$$-(\sigma_{MG} \cdot c_{MG} - \sigma_{SG} \cdot c_{SG}) \cdot l_{SG0} \cdot M.$$

Aus der Differenz der Werte E1 und E2 kann AF bestimmt werden:

$$E1 - E2 = AF = (\sigma_{MG} \cdot c_{MG} - \sigma_{SG} \cdot c_{SG}) \cdot l_{SG0} \cdot M.$$

Damit gilt für den Wert E1:

$$E1 = \sigma_{MG} \cdot l_0 \cdot c_{MG} - \frac{AF}{M}$$

und schließlich für die Konzentration $c_{MG}$ des Messgases:

$$c_{MG} = \frac{1}{\sigma_{MG} \cdot l_0} \cdot \left( E1 + \frac{AF}{M} \right) = \frac{1}{\sigma_{MG} \cdot l_0} \cdot \left( E1 + \frac{E1 - E2}{M} \right) \quad .$$

Wie bereits oben erläutert, ändert sich bei geringer Modulation des Volumenstroms des Spülgases 12 um beispielsweise 10% die von dem Spülgas bespülte Absorptionsstrecke $l_{SG}$ um denselben Prozentsatz, d. h. M = 0,1. In diesem Fall ergibt sich für die Konzentration $c_{MG}$ des Messgases:

$$c_{MG} = \frac{1}{\sigma_{MG} \cdot l_0} \cdot \left( 11 \cdot E1 - 10 \cdot E2 \right) \quad .$$

## Patentansprüche

1.  Prozess-Gasanalysator zur Analyse eines in einem Anlagenteil (1) geführten Prozessgases (2),

    - mit einer Lichtquelle (7), deren Licht (9) nach Durchstrahlen des Anlagenteils (1) mit dem Prozessgas (2) von einem Detektor (8) detektiert und in einer nachgeordneten Auswerteeinrichtung (18) hinsichtlich der Absorption in dem Prozessgas (2) zu einem Analysenergebnis (19) ausgewertet wird, und
    - mit einer Spüleinrichtung, die zwischen der Lichtquelle (7) und dem Anlagenteil (1) sowie zwischen dem Detektor (8) und dem Anlagenteil (1) jeweils eine zu dem Inneren des Anlagenteils (1) hin offene und mit einem Spülgas (12) beströmte Kammer (10, 11) aufweist, so dass das Licht (9) eine Gesamtabsorptionsstrecke bestehend aus einer von dem Spülgas (12) bespülten Absorptionsstrecke und einer Messstrecke in dem Prozessgas (2) durchläuft,
    - wobei die Spüleinrichtung Mittel (13, 16) zur Veränderung des Volumenstroms des Spülgases (12) aufweist und die Auswerteeinrichtung (18) dazu ausgebildet ist, anhand einer von der Veränderung des Volumenstroms hervorgerufenen Änderung der detektierten Absorption den Einfluss der von dem Spülgas (12) bespülten Absorptionsstrecke auf das Analysenergebnis (19) zu bestimmen und aus dem Analysenergebnis (19) zu entfernen,

    **dadurch gekennzeichnet,**

    - **dass** die Mittel (13, 16) dazu ausgebildet sind, den Volumenstrom des Spülgases (12) periodisch derart zu modulieren, dass sich die von dem Spülgas (12) bespülte Absorptionsstrecke um weniger als 100% ändert, und
    - **dass** die Auswerteeinrichtung (18) dazu ausgebildet ist, anhand von durch die Modulation hervorgerufenen Änderungen der detektierten Absorption den Einfluss des Spülgases (12) auf das Analysenergebnis (19) zu bestimmen und aus dem Analysenergebnis (19) zu entfernen.

2.  Prozess-Gasanalysator nach Anspruch 1, **dadurch gekennzeichnet, dass** die Auswerteeinrichtung (17) einen Lock-in-Demodulator enthält, der die Amplitude der Änderungen der detektierten Absorption bei der Modulationsfrequenz des Volumenstromes ermittelt.

3.  Prozess-Gasanalysator nach Anspruch 1 oder 2, **dadurch gekennzeichnet, dass** die Mittel zur Modulation des Volumenstroms des Spülgases (12) ein drehzahlvariables Gebläse (13) umfassen.

4.  Prozess-Gasanalysator nach Anspruch 1 oder 2, **dadurch gekennzeichnet, dass** die Mittel zur Modulation des Volumenstroms des Spülgases (12) ein steuerbares Regelventil (16) in der Spülgaszufuhr zu den Kammern (10, 11) aufweisen.

**5.** Verfahren zur Analyse eines in einem Anlagenteil (1) geführten Prozessgases (2),

- wobei das Licht (9) einer Lichtquelle (7) nach Durchstrahlen des Anlagenteils (1) mit dem Prozessgas (2) mittels eines Detektors (8) detektiert und in einer nachgeordneten Auswerteeinrichtung (18) hinsichtlich der Absorption in dem Prozessgas (2) zu einem Analysenergebnis (19) ausgewertet wird,
- wobei zwischen der Lichtquelle (7) und dem Anlagenteil (1) sowie zwischen dem Detektor (8) und dem Anlagenteil (1) vorhandene und zu dem Inneren des Anlagenteils (1) hin offene Kammern (10, 11) mit einem Spülgas (12) beströmt werden, so dass das Licht (9) eine Gesamtabsorptionsstrecke bestehend aus einer von dem Spülgas (12) bespülten Absorptionsstrecke und einer Messstrecke in dem Prozessgas (2) durchläuft, und
- wobei der Volumenstrom des Spülgases (12) verändert wird und anhand einer von der Veränderung des Volumenstroms hervorgerufenen Änderungen der detektierten Absorption der Einfluss der von dem Spülgas (12) bespülten Absorptionsstrecke auf das Analysenergebnis (19) bestimmt und aus dem Analysenergebnis (19) entfernt wird,

**dadurch gekennzeichnet,**

- **dass** der Volumenstrom des Spülgases (12) derart periodisch moduliert wird, dass sich die von dem Spülgas (12) bespülte Absorptionsstrecke um weniger als 100% ändert, und
- **dass** anhand von durch die Modulation hervorgerufenen Änderungen der detektierten Absorption der Einfluss des Spülgases (12) auf das Analysenergebnis (19) bestimmt und aus dem Analysenergebnis (19) entfernt wird.

**6.** Verfahren nach Anspruch 5, **dadurch gekennzeichnet, dass** die Amplitude der Änderungen der detektierten Absorption bei der Modulationsfrequenz des Volumenstromes durch Lock-in-Demodulation ermittelt wird.

**7.** Verfahren nach Anspruch 5 oder 6, **dadurch gekennzeichnet, dass** der Volumenstrom des Spülgases (12) mittels eines drehzahlvariablen Gebläses (13) moduliert wird.

**8.** Verfahren nach Anspruch 5 oder 6, **dadurch gekennzeichnet, dass** der Volumenstrom des Spülgases (12) mittels eines steuerbaren Regelventils (16) in der Spülgaszufuhr moduliert wird.

**Claims**

**1.** Process gas analyser for analysing a process gas (2) carried in a plant section (1),

- having a light source (7), the light (9) from which passes through the plant section (1) with the process gas (2) and is then detected by a detector (8) and evaluated in respect of the absorption in the process gas (2) in an evaluation unit (18) arranged downstream to produce an analysis result (19), and
- having a purging facility which in each case between the light source (7) and the plant section (1) and also between the detector (8) and the plant section (1) has a chamber (10, 11) which is open towards the interior of the plant section (1) and flushed with a purge gas (12), so that the light (9) passes through a total absorption path consisting of an absorption path, which is flushed with the purge gas (12), and a measuring path in the process gas (2),
- wherein the purging facility has means (13, 16) for the modification of the volume flow rate of the purge gas (12) and the evaluation unit (18) is designed in order to determine the effect of the absorption path flushed with the purge gas (12) on the analysis result (19) on the basis of a change in the detected absorption caused by the modification of the volume flow rate and to remove said effect from the analysis result (19),

**characterised in that**

- the means (13, 16) are designed to modulate the volume flow rate of the purge gas (12) periodically, such that the absorption path flushed with the purge gas (12) changes by less than 100%, and
- the evaluation unit (18) is designed in order to determine the effect of the purge gas (12) on the analysis result (19) on the basis of changes in the detected absorption caused by the modulation and to remove said effect from the analysis result (19).

**2.** Process gas analyser according to claim 1, **characterised in that** the evaluation unit (17) contains a lock-in demodulator which ascertains the amplitude of the changes in the detected absorption at the modulation frequency of the volume flow rate.

3. Process gas analyser according to claim 1 or 2, **characterised in that** the means for modulating the volume flow rate of the purge gas (12) comprise a variable-speed fan (13).

4. Process gas analyser according to claim 1 or 2, **characterised in that** the means for modulating the volume flow rate of the purge gas (12) comprise a controllable regulator valve (16) in the purge gas feed system to the chambers (10, 11).

5. Method for analysing a process gas (2) carried in a plant section (1),

   - wherein the light (9) from a light source (7) is detected by means of a detector (8) after passing through the plant section (1) with the process gas (2) and is evaluated in respect of the absorption in the process gas (2) in an evaluation unit (18) arranged downstream to produce an analysis result (19),
   - wherein chambers (10, 11) present between the light source (7) and the plant section (1) and also between the detector (8) and the plant section (1) and open towards the interior of the plant section (1) are flushed with a purge gas (12), so that the light (9) passes through a total absorption path consisting of an absorption path, which is flushed with a purge gas (12), and a measuring path in the process gas (2),
   - wherein the volume flow rate of the purge gas (12) is modified and the effect of the absorption path flushed with the purge gas (12) on the analysis result (19) is determined on the basis of a change in the detected absorption caused by the modification of the volume flow rate and said effect is removed from the analysis result (19),

   **characterised in that**

   - the volume flow rate of the purge gas (12) is periodically modulated, such that the absorption path flushed with the purge gas (12) changes by less than 100%, and
   - the effect of the purge gas (12) on the analysis result (19) is determined on the basis of changes in the detected absorption caused by the modulation and said effect is removed from the analysis result (19).

6. Method according to claim 5, **characterised in that** the amplitude of the changes in the detected absorption is ascertained at the modulation frequency of the volume flow rate by means of lock-in demodulation.

7. Method according to claim 5 or 6, **characterised in that** the volume flow rate of the purge gas (12) is modulated by means of a variable-speed fan (13).

8. Method according to claim 5 or 6, **characterised in that** the volume flow rate of the purge gas (12) is modulated by means of a controllable regulator valve (16) in the purge gas feed system.

**Revendications**

1. Analyseur de gaz de processus pour l'analyse d'un gaz (2) de processus passant dans une partie (1) d'installation,

   - comprenant une source (7) lumineuse, dont on détecte, par un détecteur (8), la lumière (9) après qu'elle a traversé la partie (1) d'installation ayant le gaz (2) de processus et dont on exploite, en un résultat (19) de l'analyse, dans un dispositif (18) d'exploitation en aval, l'absorption dans le gaz (2) de processus et
   - comprenant un dispositif de balayage, qui a, entre la source (7) lumineuse et la partie (1) de l'installation ainsi qu'entre le détecteur (8) et la partie (1) d'installation, respectivement une chambre (10, 11) ouverte vers l'intérieur de la partie (1) d'installation et balayée par un gaz (12) d'entraînement, de sorte que la lumière (9) traverse une section d'absorption globale constituée d'une section d'absorption balayée par le gaz (12) d'entraînement et d'une section de mesure dans le gaz (2) de processus,
   - dans lequel le dispositif de balayage a des moyens (13, 16) pour faire varier le courant en volume du gaz (12) d'entraînement et le dispositif (18) d'exploitation est constitué de manière à déterminer, à l'aide d'une variation provoquée par la variation du courant en volume, de l'absorption détectée, l'influence de la section d'absorption balayée par le gaz (12) d'entraînement sur le résultat (19) de l'analyse et à l'éliminer du résultat (19) de l'analyse,

   **caractérisé**

- **en ce que** les moyens (13, 16) sont constitués pour moduler périodiquement le courant en volume du gaz (12) d'entraînement, de manière à faire varier, de moins de 100%, la section d'absorption balayée par le gaz (12) d'entraînement et

- **en ce que** le dispositif (18) d'exploitation est constitué pour, à l'aide des variations, provoquées par la modulation, de l'absorption détectée, déterminer l'influence du gaz (12) d'entraînement sur le résultat (19) de l'analyse et l'éliminer du résultat (19) de l'analyse.

2. Analyseur de gaz de processus suivant la revendication 1, **caractérisé en ce que** le dispositif (17) d'exploitation comporte un démodulateur lock-in, qui détermine l'amplitude des variations de l'absorption détectée à la fréquence de modulation du courant en volume.

3. Analyseur de gaz de processus suivant la revendication 1 ou 2, **caractérisé en ce que** les moyens de modulation du courant en volume du gaz (12) d'entraînement comprennent une soufflante (13) à vitesse de rotation variable.

4. Analyseur de gaz de processus suivant la revendication 1 ou 2, **caractérisé en ce que** les moyens de modulation du courant en volume du gaz (12) d'entraînement comportent une vanne (16) de régulation réglable dans l'apport du gaz d'entraînement aux chambres (10, 11).

5. Procédé d'analyse d'un gaz (2) de processus passant dans une partie (1) d'installation,

- dans lequel on détecte, au moyen d'un détecteur (8) la lumière (9) d'une source (7) lumineuse, après qu'elle a traversé la partie (1) d'installation ayant le gaz (2) de processus et, dans un dispositif (18) d'exploitation en aval, on exploite, en un résultat (19) de l'analyse, son absorption dans le gaz (2) de processus,

- dans lequel on alimente, en un gaz (12) d'entraînement, des chambres (10, 11) présentes entre la source (7) lumineuse et la partie (1) d'installation, ainsi qu'entre le détecteur (8) et la partie (1) d'installation et ouvertes vers l'intérieur de la partie (1) d'installation, de manière à faire passer la lumière (9) dans le gaz de processus dans une section d'absorption globale constituée d'une section d'absorption balayée par le gaz (2) de processus et d'une section de mesure et

- dans lequel on fait varier le courant en volume du gaz (12) d'entraînement et, à l'aide d'une variation, provoquée par la variation du courant en volume, de l'absorption détectée, on détermine l'influence de la section d'absorption balayée par le gaz (12) d'entraînement sur le résultat (19) de l'analyse et on l'élimine du résultat (19) de l'analyse,

**caractérisé**

- **en ce que** l'on module périodiquement le courant en volume du gaz (12) d'entraînement, de manière à faire varier, de moins de 100%, la section d'absorption balayée par le gaz (12) d'entraînement et

- **en ce que**, à l'aide de variations provoquées par la modulation de l'absorption détectée, on détermine l'influence du gaz (12) d'entraînement sur le résultat (19) de l'analyse et on l'élimine du résultat (19) de l'analyse.

6. Procédé suivant la revendication 5, **caractérisé en ce que** l'on détermine, par une démodulation lock-in, l'amplitude des variations de l'absorption détectée à la fréquence de modulation du courant en volume.

7. Procédé suivant la revendication 5 ou 6, **caractérisé en ce que** l'on module le courant en volume du gaz (12) d'entraînement au moyen d'une soufflante (13) à vitesse de rotation variable.

8. Procédé suivant la revendication 5 ou 6, **caractérisé en ce que** l'on module le courant en volume du gaz (12) d'entraînement au moyen d'une vanne (16) de réglage pouvant être commandée.

FIG. 1

FIG. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120236323 A1
- DE 102013213730 A1
- EP 1693665 A1
- DE 102012223874 B3